# EUROPÄISCHE PATENTANMELDUNG

(11) **EP 1 503 265 A1**
(43) Veröffentlichungstag der Anmeldung: **02.02.2005**
(21) Anmeldenummer: 04017909.5
(22) Anmeldetag: 29.07.2004
(51) Int. Cl.: G05G 1/14

(54) **Fusssteuerung**

(30) Priorität: 31.07.2003 AT 12132003
(71) Anmelder: W & H Dentalwerk Bürmoos GmbH, 5111 Bürmoos (AT)
(72) Erfinder: Meingassner, Wolfgang, A-5122 Überackern (AT); Haberl-Resch, Silvia, A-5020 Salzburg (AT)

(57) **Zusammenfassung**

Die vorliegende Erfindung betrifft eine Fußsteuerung (1) zur Bedienung insbesondere medizinischer und zahnärztlicher Geräte oder Steuervorrichtungen mit zumindest einem Taster (2-5). Um Anforderungen aus unterschiedlichen Anwendungsbereichen und individuellen Wünschen der Benutzer nachkommen zu können sowie die Bedienung einfacher und sicherer zu gestalten wird vorgeschlagen, die Verbindung zwischen zumindest zwei Tastern (2 - 5) aus einem vom Gehäuse (23, 33, 43, 53) oder vom Gehäuse (23, 33, 43, 53) und der Basisplatte (7) geformten Steg (32, 42, 52) zu bilden, dessen Breite maximal gleich der Länge des Tasters (2 - 5) ist und bei zumindest einem Taster (2-5) den Tasterdeckel (21, 31, 41, 51) und/oder einen Teil des Tastergehäuses (23, 33, 43, 53) und/oder einen Teil des Schalt-/Steuerelements und/oder der Leiterbahn lösbar mit der Fußsteuerung (1) zu verbinden. Weiters wird die Drehachse (26) zumindest eines Tasters (2 - 5) ungefähr in der Verlängerung der Achse (105) des Unterschenkels (106) des Beins, das die Fußsteuerung (1) bedient, angeordnet und die zur Betätigung des Tasters (2 - 5) aufzuwendende Druckkraft kann über lösbare Federn (80, 81) unter dem Tasterdeckel (21, 31, 41, 51), deren Position veränderbar ist, variiert werden.

## Beschreibung

Die vorliegende Erfindung betrifft eine Fußsteuerung zur Bedienung insbesondere medizinischer und zahnärztlicher Geräte oder Steuervorrichtungen mit einer Basisplatte, einem darauf angeordneten Gehäuse und zumindest einem Taster zur Bedienung von einem oder mehreren Steuer-/Schaltelement(en).

Bei derartigen Fußsteuerungen sind um einen zentralen und größer ausgebildeten Taster ein oder mehrere, häufig kleiner ausgebildete Taster seitlich angeordnet, siehe zum Beispiel EP 1 243 223 A2 (Figur 1) und EP 789 929 B1 (Figur 1).

Nachteilig dabei ist die geringe Flexibilität die diese Fußsteuerungen den Benutzern verschiedener Anwendungsbereiche eröffnen: In Bereichen in denen grobes Schuhwerk getragen wird, beispielsweise bei unfallchirurgischen Eingriffen wo zwecks einfacher Desinfektion oftmals Gummischuhe verwendet werden, ist die taktile Wahrnehmung der Anwender merklich verringert, so daß die Bedienung der kleinen Taster oftmals schwer zu kontrollieren ist bzw. die Gefahr besteht unbeabsichtigt auf mehrere Taster gleichzeitig zu treten.

In bestimmten Anwendungsgebieten, zum Beispiel im Hals-Nasen-Ohren Bereich, genügt es, wenn mit der Fußsteuerung ein Parameter eines gerade eingesetzten Instruments, etwa die Drehzahl eines Bohrers oder die Geschwindigkeit einer Säge, verändert werden kann, so daß Anwender in diesem Fall eine Fußsteuerung mit nur einem Taster bevorzugen. Hingegen benötigen Anwender zum Beispiel im Bereich der Augenchirurgie oder der Implantologie, wo komplexe Steuergeräte die Auswahl von verschiedenen Betriebsprogrammen und die Einstellung einer Vielzahl von Parametern ermöglichen, Fußsteuerungen über die möglichst viele Einstellungen am Steuergerät vorgenommen werden können, um die Berührung des unsterilen Steuergeräts zu vermeiden und um sich möglichst selten von der Operationsstelle abwenden zu müssen.

Benutzen mehrere Anwender eine Fußsteuerung, so bestehen oftmals auch sehr unterschiedliche persönliche Vorlieben wie viele Taster benutzt werden oder wie groß die zum Bedienen der Taster aufzuwendende Kraft ist.

Die in den beiden Schriften EP '223 und EP '929 dargestellten Fußsteuerungen bieten keine Möglichkeit auf die unterschiedlichen Anforderungen und Präferenzen der Anwender einzugehen. Um den Wünschen der Benutzer dennoch nachzukommen, erzeugen manche Hersteller eine größere Anzahl unterschiedlicher Fußsteuerungen, was jedoch einen erhöhten produktions- und verwaltungstechnischen Aufwand verursacht.

Der vorliegenden Erfindung liegt daher die Aufgabe zugrunde eine verbesserte Fußsteuerung derart zu gestalten, daß die Bedienung der Fußsteuerung sicherer und einfacher wird und insbesondere der Anwender Anpassungen an Erfordernisse aus unterschiedlichen Anwendungsgebieten oder an persönliche Präferenzen in unkomplizierter Weise und ohne großen zeitlichen Aufwand selbständig durchführen kann.

Diese Aufgabe wird gemäß der vorliegenden Erfindung durch eine Fußsteuerung mit den Merkmalen des Anspruchs 1, 2, 8 und 10 gelöst. Besonders vorteilhafte Ausführungsformen sind in den entsprechenden Unteransprüchen angeführt.

Bei der erfindungsgemäßen Fußsteuerung gemäß Anspruch 1 erleichtert die Trennung der Taster mittels eines Stegs, dessen Breite geringer ist als die Länge der Taster, die taktile Wahrnehmung der einzelnen Taster durch den Benutzer. Eine eindeutige Wahrnehmung der Trennung ist bei unterschiedlich großen Tastern gegeben, solange die Breite des Stegs maximal der Länge des kleineren Tasters entspricht.

Die Länge des Tasters ist dabei wie folgt definiert: Ist die Fußsteuerung so vor dem Anwender positioniert, daß dieser sie bestimmungsgemäß bedienen kann, so weist jeder Taster eine dem Anwender nahe und entfernte Seite (beispielsweise bei rechteckigen Tastern) bzw. einen dem Anwender nächsten und entferntesten Punkt (z.B. bei kreisförmigen Tastern) auf. Die kürzeste Verbindung zwischen diesen beiden Seiten bzw. Punkten entspricht der Länge des Tasters.

In einem Ausführungsbeispiel ist der Steg deutlich schmäler als die Länge des Tasters, bevorzugt weniger als die Hälfte der Länge des Tasters, besonders bevorzugt weniger als ein Viertel der Länge des Tasters.

Sind in einem Ausführungsbeispiel die Stege, die die Taster verbinden, asymmetrisch ausgebildet, zum Beispiel sich verjüngend von einem größeren Taster zu einem kleineren Taster, so darf, um eine für den Anwender ausreichende Unterscheidung der Taster zu gewährleisten, die geringste Breite des Stegs maximal der Länge des kleineren Tasters entsprechen.

Um dem Anwender das Auffinden der Taster zu erleichtern umgibt in einem weiteren Ausführungsbeispiel das Gehäuse die einzelnen Taster (Tasterdeckel), so daß um jeden Taster ein durch das Gehäuse gebildeter Rand vorliegt. Um dem Anwender auch in diesem Fall eine ausreichende Unterscheidung der die Taster umgebenden Gehäuse zu garantieren, gelten die oben beschriebenen Merkmale in gleicher Weise auch für die die Taster umgebenden Gehäuse.

In einem weiteren Ausführungsbeispiel sind die durch Stege getrennten Taster und/oder die diese Taster umgebenden Gehäuseabschnitte unterschiedlich hoch, so daß die Unterscheidung der einzelnen Taster weiter erleichtert wird.

Durch die lösbare Verbindung des Tasterdeckels und/oder eines Teils des Tastergehäuses und/oder des Schalt-/Steuerelements und/oder der Leiterbahn mit der Fußsteuerung gemäß Anspruch 2 können der Hersteller oder der Anwender ihren Vorstellungen entsprechend einen oder mehrere Taster inaktivieren, so daß der Taster kein Signal abgibt.

In einem ersten Ausführungsbeispiel ist ausschließlich der Tasterdeckel lösbar mit der Fußsteuerung verbunden. Auf der dem Schalterelement zugewandten Seite des Tasterdeckels befindet sich ein Teil des Schalt-/Steuerelements, z.B. ein Fortsatz, der bei Krafteinwirkung auf den Taster einen mechanischen Schalter einschaltet oder ein Magnet. Die Entfernung des Tasterdeckels verhindert somit die Aktivierung des Schalters. Bevorzugt wird der Tasterdeckel durch einen Blindstopfen (Deckel ohne Fortsatz oder Magnet) ersetzt, um das Eindringen von Verunreinigungen in den Taster und den Schalter zu vermeiden.

In einem zweiten Ausführungsbeispiel wird der Tasterdeckel zusammen mit einem weiteren Teil des Tasters und des Schalt-/Steuerelements und/oder der Leiterbahn von der Fußsteuerung getrennt und bevorzugt durch einen Blindstopfen, der die gleiche Form wie der Taster hat, dem jedoch der entfernte Teil des Schalt-/Steuerelements fehlt, ersetzt.

In einem dritten Ausführungsbeispiel wird der gesamte Taster einschließlich der zugehörigen Gehäuseteile und der darin enthaltenen. Schalt-/Steuerelemente und/oder der Leiterbahnen, bevorzugt auch mit dem Steg, entfernt, so daß an der Fußsteuerung nur aktive Taster zur Verfügung stehen und der Anwender nicht zwischen aktiven und z.B. durch Blindstopfen inaktiven Tastern unterscheiden muß.

Der Vorteil der drei beschriebenen Ausführungsbeispiele gegenüber der Inaktivierung eines oder mehrere Taster z.B. über die Software der Steuervorrichtung oder einen Sperrmechanismus, der beispielsweise die Bewegung des Tasterdeckels verhindert, liegt darin, daß dem Anwender das Auffinden der verbliebenen aktiven Taster aufgrund der in ihrer Höhe reduzierten oder vollkommenen entfernten inaktiven Tastern stark erleichtert wird und ein versehentliches Bedienen von inaktiven Tastern oder ein Hängenbleiben daran bei entfernten Tastern vermieden wird bzw. bei in ihrer Höhe reduzierten Tastern die Möglichkeit dazu verringert ist.

Als lösbare Verbindungen können sämtliche gemäß dem Stand der Technik bekannte Verbindungen eingesetzt werden, bevorzugt solche, für deren Bedienung man keine Werkzeuge benötigt, z.B. Steck-, Klemm-, Bajonett- oder Schraubverbindung.

In einem weiteren Ausführungsbeispiel ist ein Taster im Vergleich zu den anderen Tastern größer ausgebildet, bevorzugt ist dieser Taster zentral angeordnet und dient der stufenlosen Steuerung eines Betriebsparameters (z.B. der Drehzahl), während die peripher angeordneten, kleineren Taster zur Auswahl von Programmen, zur Einstellung von Betriebsparametem oder zum Ein-/Ausschalten von Zusatzfunktionen (z.B. Zufuhr eines Kühlmediums) verwendet werden.

Da das Funktionsprinzip der Fußsteuerung, geeignete Schalt-/Steuerelemente (wie zum Beispiel Hall-Sensoren, Potentiometer, mechanische Taster, Reed-Sensoren, Membranschalter usw.) und deren Wirkungsweise, die Weiterleitung und Verarbeitung der von der Fußsteuerung erzeugten Signale sowie der Anschluß an elektrische bzw. elektronische Schaltsysteme von insbesondere medizinischen und zahnärztlichen Geräte oder Steuervorrichtungen bei der erfindungsgemäßen Fußsteuerung dem Stand der Technik entsprechen und einem Fachmann bekannt sind, wird darauf nicht weiter eingegangen.

Bei der erfindungsgemäßen Fußsteuerung nach Anspruch 8 wird durch die Anordnung der Drehachse in jener Hälfte des Tasters, bevorzugt in jenem Viertel des Tasters, die/das der Ferse des Anwenders am nächsten ist, besonders bevorzugt im Bereich der Verlängerung der Längsachse des Unterschenkels und unterhalb des Sprunggelenks des Anwenders des Beins, das die Fußsteuerung bedient, eine besonders hohe Leichtgängigkeit und damit eine geringe Ermüdung bei längerer Bedienung erreicht. Der Bereich des Sprunggelenks des Beins ist insbesondere deshalb von großem Vorteil, da dadurch die Drehachse des Fußes um das Bein und die Drehachse des Tasterdeckels parallel angeordnet sind, wodurch eine maximale Kraftübertragung und Hebelwirkung entstehen.

Bei der erfindungsgemäßen Fußsteuerung nach Anspruch 10 können Anwender gemäß ihren Präferenzen und entsprechend ihrer Fußgröße die zum Betätigen des Tasters benötigte Kraft variieren. Dazu wird der Tasterdeckel von der Fußsteuerung gelöst, um Zugang zu zumindest einer darunter lösbar angeordneten Feder, die zum Vorspannen des Tasters bzw. zur Rückstellung in seine Ausgangsposition dient, zu erhalten.

In einem Ausführungsbeispiel stehen dem Anwender mehrere Federn mit unterschiedlichen Federkraft zur Verfügung, so daß durch den Tausch der Federn die zum Betätigen des Tasters benötigte Kraft verändert werden kann. Die Federn werden über Steckverbindungen, zum Beispiel Klammern, die als Teil des Tasterdeckels und/oder des Tastergehäuses ausgeführt sind und die Enden der Federn aufnehmen, befestigt.

In einem weiteren Ausführungsbeispiel kann der Anwender die zumindest eine, lösbar angeordnete Feder in mehreren, bevorzugt drei bis fünf, Aufnahmen fixieren, wobei jede Aufnahme in unterschiedlicher Entfernung von der Drehachse des Tasters angeordnet ist. Die Aufnahmen können bevorzugt durch Vertiefungen im Tastergehäuse gebildet sein. Je näher die zumindest eine Feder zur Drehachse positioniert ist, um so geringer ist die zum Niederdrücken des Tasterdeckels benötigte Kraft. Unter Drehachse ist erfindungsgemäß nicht nur eine physisch vorhandene Achse, z.B. in Form eines Bolzens, zu verstehen, sondern allgemein jener Bereich des Tasterdeckels, der bei Betätigung des Tasters keine bzw. nur eine sehr geringe Winkelbewegung durchläuft. Vorteilhaft ist bei diesem Ausführungsbeispiel, daß der Anwender kein Set von Federn aufbewahren muß.

Eine Kombination der beiden Ausführungsbeispiele führt zu einer besonders feinstufigen Variation der zum Betätigen des Tasters benötigte Kraft.

Die Erfindung wird nachfolgend anhand bevorzugter Ausführungsbeispiele und Bezug nehmend auf die beigefügten Zeichnungen erläutert:
Figur 1 zeigt eine erfindungsgemäße Fußsteuerung in perspektivischer Ansicht.
Figur 2 zeigt eine erfindungsgemäße Fußsteuerung mit drei seitlichen Tastern in Explosionsdarstellung.
Figur 3 zeigt ein alternatives Ausführungsbeispiel der erfindungsgemäßen Fußsteuerung mit vollkommen von der Fußsteuerung lösbaren Tastem.
Figur 4 zeigt in schematischer Ansicht eine erfindungsgemäße Fußsteuerung mit der Drehachse des Tasterdeckels in der Verlängerung der Achse des Unterschenkels.

In allen Figuren sind gleiche Bauteile mit den gleichen Ziffern beschriftet.

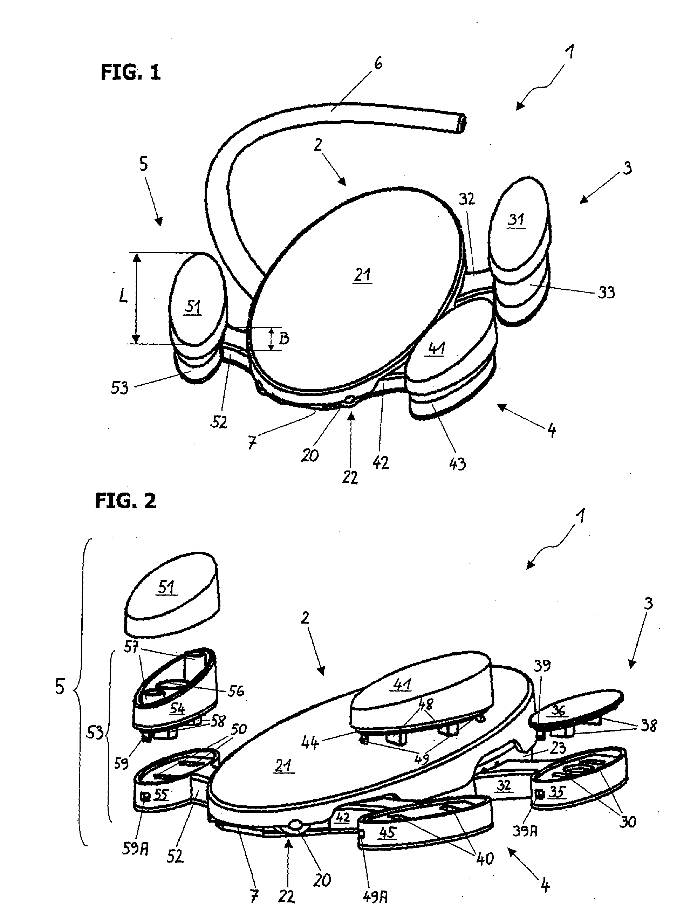

Die in Figur 1 abgebildete Fußsteuerung 1 weist vier Taster 2 - 5 auf, wovon Taster 2 als zentraler, großer Taster ausgebildet ist, der von den kleineren Tastern 3 - 5 umgeben ist. Jeder der vier Taster 2 - 5 setzt sich aus einem Tasterdeckel 21, 31, 41, 51 und einem Tastergehäuse 23, 33, 43, 53 zusammen. Die peripheren Taster 3 - 5 sind über jeweils einen Steg 32, 42, 52 mit dem Tastergehäuse 23 (siehe Figur 2) des Tasters 2 verbunden. Um dem Benutzer die Wahrnehmung der einzelnen Taster zu erleichtern, sind die Stege 32, 42, 52 schmaler als die Taster 3 - 5 ausgebildet, ihre Breite B beträgt etwa ein Viertel der Länge L der Taster 3 - 5. Weiters sind die Taster 3 - 5 höher als der Taster 2, was dem Anwender das Erkennen und Unterscheiden der Taster 3 - 5 zusätzlich vereinfacht.

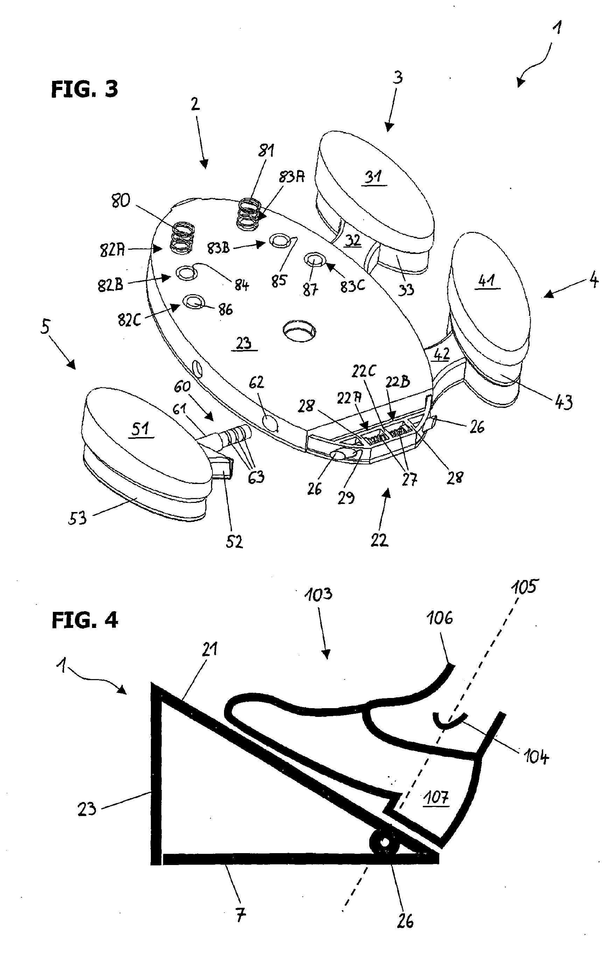

Alle Tastergehäuse 23, 33, 43, 53 und die Stege 32, 42, 52 sind auf der Bodenplatte 7 angeordnet. Der seitlich am Tastergehäuse 23 angebrachte Bügel 6 dient der einfachen und komfortablen Änderung des Stellplatzes der Fußsteuerung 1 und kann bei Bedienung der Fußsteuerung 1 nach vorne geklappt werden. Über die Verriegelung 22 kann der Tasterdeckel 21 vom Tastergehäuse 23 getrennt werden. Die Verriegelung 22 besteht aus zwei identischen Kammern 22A und 22B, die durch eine Wand 22C voneinander getrennt sind und jeweils einen Führungssteg 28 mit einer Öffnung aufweisen (siehe Figur 3). Durch diese Öffnung ragt ein Bolzen 26, der durch eine weitere Öffnung 29 des Tastergehäuses 23 nach außen tritt. Bolzen 26 wird im Inneren der Kammer 22A bzw. 22B durch eine Feder 27 vorgespannt, so daß sein Endabschnitt so weit von der Seitenwand des Tastergehäuses 23 absteht, daß dem Anwender ein unkompliziertes Lösen bzw. Anbringen des Tasterdeckels 21 möglich ist. Dies erfolgt durch Drücken der beiden Bolzen 26 entgegen der Wirkrichtung der Federn 27, wodurch die Endabschnitte der Bolzen 26 in die Kammern 22A, 22B verschoben werden. Nun kann der Tasterdeckel 21 auf das Tastergehäuse 23 aufgesetzt und die Bolzen 26 können losgelassen werden. Tasterdeckel 21 weist an seinen Seiten zwei Öffnungen 20 auf (siehe Figur 1), die, nachdem der Tasterdeckel 21 auf das Tastergehäuse 23 aufgesetzt worden ist, über den Öffnungen 29 liegen. Die Bolzen 26 werden durch die Federn 27 nach außen verschoben und ragen durch beide Öffnungen 20 und 29, wodurch der Tasterdeckel 21 am Gehäuse 23 fixiert ist. Gleichzeitig dienen die Bolzen 26 auch als Drehachse für den Tasterdeckel 21.

Wie aus Figur 2 erkennbar ist, besteht jedes Tastergehäuse 33, 43, 53 der Fußtaster 3 - 5 aus einem Tastergehäuse-Oberteil 44, 54 (bei Taster 3 nicht dargestellt) und einem Tastergehäuse-Unterteil 35, 45, 55. Der detaillierte Aufbau ist exemplarisch an Taster 5 zu erkennen: Über Steg 52 ist das Tastergehäuse-Unterteil 55 mit dem Gehäuse 23 verbunden. Mittels einer lösbaren Steckverbindung, bestehend aus den Öffnungen 50 und den Fortsätzen 58, sowie einer lösbaren Schnapphakenverbindung 59, 59A ist das Tastergehäuse-Oberteil 54 an dem Tastergehäuse-Unterteil 55 befestigt. Form und Durchmesser der beiden Gehäuseteile 54 und 55 sind aufeinander abgestimmt, so daß eine möglichst dichte Verbindung entsteht. Form und Durchmesser von Tasterdeckel 51 sind derart bemessen, daß der Tasterdeckel 51 über das Tastergehäuse-Oberteil 54 geschoben werden kann. Zusammengefügt sind der Tasterdeckel 51 und das Tastergehäuse-Oberteil 54 über eine Schraubverbindung mit hohlen, zylinderförmigen Aufnahmen 57 im Tastergehäuse-Oberteil 54, in denen jeweils eine Feder und eine Schraube gelagert sind. An der Innenseite des Tasterdeckels 51 befindet sich ein Konus mit Innengewinde zur Aufnahme der Schraube. Die in den Aufnahmen 57 enthaltenen Federn spannen den Tasterdeckel 51 gegen das Tastergehäuse-Oberteil 54 vor. Bei Krafteinwirkung auf den Tasterdeckel 51 kommt es gegen die Kraftwirkung der Federn zu einer Relativbewegung des Tasterdeckels 51 gegen das Tastergehäuse-Oberteil 54. An der Innenseite des Tasterdeckels 51 ist ein Schalt-/Steuerelement (nicht dargestellt), z.B. ein Permanentmagnet, angebracht. Durch die Relativbewegung des Tasterdeckels 51 kommt es zu einer Annäherung des Schalt-/Steuerelements an einen Sensor 56, z.B. einen Hall-Sensor, im Tastergehäuse-Oberteil 54. Der Hall-Sensor dedektiert das Magnetfeld bzw. die Veränderung der Magnetfeldstärke und sendet ein Signal über Leiterbahnen (nicht dargestellt) vom Taster 5 über den Steg 52 zur Platine im Tastergehäuse 23. Mit Beendigung der Krafteinwirkung wird der Tasterdeckel 51 durch die Federn wieder in seine Ausgangsposition bewegt.

Aufgrund des mehrteiligen Aufbaus der Taster 3, 4, 5 und der lösbaren Verbindungen zwischen den einzelnen Tasterteilen kann der Anwender zur Inaktivierung eines oder mehrere Taster 3, 4, 5 die Tasterdeckel 31, 41, 51 und/oder die Tastergehäuseoberteile 44, 54 von der Fußsteuerung 1 lösen und entweder die lösbar darin angeordneten Schalt-/Steuerelemente und/oder Leiterbahnen oder bevorzugt das gesamte Tasterteil (Tasterdeckel 31, 41, 51, Tastergehäuseoberteile 44, 54) mit den daran angeordneten Schalt-/Steuerelementen und/oder Leiterbahnen entfernen.

Taster 4 in Figur 2 stellt eine alternative Ausführungsform dar: Im Gegensatz zu Taster 5 sind der Tasterdeckel 41 und das Tastergehäuse-Oberteil 44 hier für den Anwender unlösbar miteinander verbunden. Benötigt der Anwender Taster 4 nicht, so kann er den Tasterdeckel 41 und das Tastergehäuse-Oberteil 44 mit den darin enthaltenen Schalt-/Steuerelement als Einheit vom Tastergehäuse-Unterteil 45 trennen und so den Taster 4 inaktivieren. Die Verbindung zwischen dem Tastergehäuse-Oberteil 44 und dem Tastergehäuse-Unterteil 45 wird wie bei Taster 5 über eine lösbare Steckverbindung 40, 48 und eine lösbare Schnapphakenverbindung 49, 49A gebildet. Das am Tastergehäuse 23 verbleibende Tastergehäuse-Unterteil 45 und der Steg 42 liegen unterhalb des Tasterdeckels 21, womit die Bedienung der Fußsteuerung erleichtert und ein ungewolltes Hängenbleiben oder Berühren des inaktiven Tasters 4 vermieden wird.

Das Eindringen von Schmutzpartikeln in die Taster 3, 4, 5 und damit in die gesamte Fußsteuerung 1 bei entferntem Tasterdeckel 31, 41, 51 oder Tastergehäuse-Oberteil 34, 44, 54 kann durch Anbringen eines Blindstopfens 36, exemplarisch an Taster 3 in Figur 2 dargestellt, vermieden werden. Der Blindstopfen 36 ist mit der selben lösbaren Steckverbindung 30, 38 und lösbaren Schnapphakenverbindung 39, 39A wie die Tastergehäuse-Oberteile 44, 54 ausgestattet, so daß der Anwender in einfacher Weise Tasterdeckel 31 und Tastergehäuse-Oberteil (nicht dargestellt) durch Blindstopfen 36 ersetzen kann.

Ein weiteres alternatives Ausführungsbeispiel ist in Figur 3 abgebildet: Hier können die Taster 3, 4, 5 mit ihren jeweiligen Stegen 32, 42, 52 und den darin angeordneten Schalt/Steuerelementen und Leiterbahnen vom Tastergehäuse 23 gelöst werden. Die Verbindung zwischen den Tastern 3, 4, 5 und dem Tastergehäuse 23 erfolgt über eine Steckverbindung 60, wie sie exemplarisch an Taster 5 dargestellt ist. Steckverbindung 60 besteht aus einem Fortsatz 61 an Steg 52, in dem die Leiterbahnen zur Signalleitung und Stromversorgung verlaufen und in drei Kontakten 63 enden. An der Seitenwand des Gehäuses 23 befindet sich eine Öffnung 62 zur Aufnahme des Fortsatzes 61. In zusammengesetztem Zustand stellen die drei Kontakte 63 die Verbindung zur Leiterbahn im Tastergehäuse 23 sicher. Bei abgenommenem Taster 5 kann Öffnung 62 wiederum durch einen Blindstopfen verschlossen werden.

Um die zum Betätigen des Tasters benötigte Kraft variieren zu können sind am Tastergehäuse 23 mehrere Aufnahmen 82A-C für die Feder 80 bzw. 83A-C für die Feder 81 vorgesehen. Jede dieser Aufnahmen 82A-C bzw. 83A-C weist eine andere Entfernung zu den Bolzen 26 (= Drehachse) des Tasters auf. Jede Aufnahme 82A-C bzw. 83A-C besteht aus einer Vertiefung 84, 85 und einem darin angeordneten Führungszapfen 86, 87. Die Federn 80, 81 sind lösbar in die Vertiefungen 84, 85 eingesetzt und können vom Anwender entsprechend seinem Wunsch versetzt werden. Eine besonders feinstufige Variation der zum Betätigen des Tasterdeckels 21 benötigte Kraft wird erreicht, wenn dem Anwender mehrere Federn mit unterschiedlichen Federkräften jedoch gleichem Durchmesser zur Verfügung stehen, so daß diese in die Aufnahmen 82A-C bzw. 83A-C eingesetzt werden können.

In Figur 4 ist in schematischer Weise eine erfindungsgemäße Fußsteuerung 1 in Seitenansicht im Längsschnitt durch den Taster 2 dargestellt. Der Fuß 103 eines Anwenders befindet sich zwecks Bedienung auf dem Tasterdeckel 21. Durch die Anordnung der Drehachse 26 in jenem Viertel des Tasterdeckels 21, das der Ferse 107 des Anwenders am nächsten ist, insbesondere in der Verlängerung der Längsachse 105 des Unterschenkels 106 und unterhalb des Sprunggelenks 104 des Anwenders, wird eine besonders hohe Leichtgängigkeit und damit eine geringe Ermüdung bei längerer Bedienung erreicht.

Die Erfindung ist nicht auf die dargestellten Ausführungsbeispiele beschränkt, sondern umfaßt alle Ausführungsmöglichkeiten, die das prinzipielle, sinngemäßen Funktionsprinzip der Erfindung nicht verändern.

## Patentansprüche

1. Fußsteuerung (1) zur Bedienung insbesondere medizinischer und zahnärztlicher Geräte oder Steuervorrichtungen mit einer Basisplatte (7), einem darauf angeordneten Gehäuse (23, 33, 43, 53) und mehr als einem Taster (2 - 5),
**dadurch gekennzeichnet, daß**
die Verbindung zwischen zumindest zwei Tastern (2 - 5) oder den diese Taster (2 - 5) umgebenden Gehäusen (23, 33, 43, 53) aus einem vom Gehäuse (23, 33, 43, 53) oder vom Gehäuse (23, 33, 43, 53) und der Basisplatte (7) geformten Steg (32, 42, 52) gebildet ist.

2. Fußsteuerung (1) zur Bedienung medizinischer, insbesondere zahnärztlicher Geräte oder Steuervorrichtungen mit einer Basisplatte (7), einem darauf angeordneten Gehäuse (23, 33, 43, 53), zumindest einem Taster (2 - 5) und einem jedem Taster (2 - 5) zugeordneten Tasterdeckel (21, 31, 41, 51), Tastergehäuse (23, 33 43, 53), Schalt-/Steuerelement und Leiterbahn oder Fußsteuerung (1) nach Anspruch 1 mit einem jedem Taster (2 - 5) zugeordneten Tasterdeckel (21, 31, 41, 51), Tastergehäuse (23, 33 43, 53), Schalt/Steuerelement und Leiterbahn,
**dadurch gekennzeichnet, daß**
bei zumindest einem Taster (2-5) der Tasterdeckel (21, 31, 41, 51) und/oder ein Teil des Tastergehäuses (23, 33, 43, 53) und/oder des Schalt-/Steuerelements und/oder der Leiterbahn lösbar mit der Fußsteuerung (1) verbunden ist.

3. Fußsteuerung (1) nach Anspruch 1 oder 2,
**dadurch gekennzeichnet, daß**
die Breite des Stegs (32, 42, 52) weniger als die Hälfte der Länge des jeweiligen Tasters (2 - 5) oder des den Taster (2 - 5) umgebenden Gehäuses (23, 33, 43, 53), bevorzugt weniger als ein Viertel der Länge des Tasters (2 - 5) oder des Gehäuses (23, 33, 43, 53) beträgt.

4. Fußsteuerung (1) nach Anspruch 2 oder 3,
**dadurch gekennzeichnet, daß**
ein Blindstopfen (36) den entfernten Tasterdeckel (21, 31, 41, 51) oder das entfernte Tastergehäuse-Oberteil (44, 54) ersetzt.

5. Fußsteuerung (1) nach einem der Ansprüche 2, 3 oder 4,
**dadurch gekennzeichnet, daß**
zumindest ein Taster (3 - 5) in seiner Gesamtheit, bevorzugt auch mit dem Steg (32, 42 52), lösbar mit der Fußsteuerung (1) verbunden ist.

6. Fußsteuerung (1) nach einem der vorhergehenden Ansprüche,
**dadurch gekennzeichnet, daß**
ein Taster größer ausgebildet ist als die restlichen Taster.

7. Fußsteuerung nach einem der vorhergehenden Ansprüche,
**dadurch gekennzeichnet, daß**
zumindest ein Taster (2 - 5) und/oder die diesen Taster (2 - 5) umgebenden Gehäuseabschnitte (44, 54, 35, 45, 55) gegenüber den anderen Tastern (2 - 5) erhöht ist/sind.

8. Fußsteuerung (1) zur Bedienung insbesondere medizinischer und zahnärztlicher Geräte oder Steuervorrichtungen mit einer Basisplatte (7), einem darauf angeordneten Gehäuse (23, 33, 43, 53) und zumindest einem Taster (2 - 5), der drehbar an einer Drehachse (26) gelagert ist oder Fußsteuerung (1) nach einem der vorhergehenden Ansprüche mit zumindest einem Taster (2 - 5), der drehbar an einer Drehachse (26) gelagert ist,
**dadurch gekennzeichnet, daß**
die Drehachse (26) zumindest eines Tasters (2 - 5) in jener Hälfte des Tasters, bevorzugt in jenem Viertel des Tasters (2 - 5), die/das der Ferse (107) des Anwenders am nächsten ist, angeordnet ist.

9. Fußsteuerung (1) nach Anspruch 8,
**dadurch gekennzeichnet, daß**
die Drehachse (26) zumindest eines Tasters (2 - 5) ungefähr in der Verlängerung der Achse (105) des Unterschenkels (106) des Beins, das die Fußsteuerung (1) bedient, angeordnet ist.

10. Fußsteuerung (1) zur Bedienung insbesondere medizinischer und zahnärztlicher Geräte oder Steuervorrichtungen mit einer Basisplatte (7), einem darauf angeordneten Gehäuse (23, 33, 43, 53) und zumindest einem Taster (2 - 5) mit einem Tasterdeckel (21, 31, 41, 51), der drehbar an einer Drehachse (26) gelagert ist und der über zumindest eine Feder (80, 81) vorgespannt ist oder Fußsteuerung (1) nach einem der vorhergehenden Ansprüche mit einem Tasterdeckel (21, 31, 41, 51), der drehbar an einer Drehachse (26) gelagert ist und der über zumindest eine Feder (80, 81) vorgespannt ist,
**dadurch gekennzeichnet, daß**
die zumindest eine Feder (80, 81) lösbar mit dem Tasterdeckel (21, 31, 41, 51) und/oder dem Tastergehäuse (23, 33, 43, 53) verbunden ist.

11. Fußsteuerung (1) nach Anspruch 10,
**dadurch gekennzeichnet, daß**
der zumindest einen Feder (80, 81) zumindest zwei Aufnahmen (82A-C, 83A-C), bevorzugt Vertiefungen (84, 85), zugeordnet sind, an denen die Feder (80, 81) lösbar mit dem Tasterdeckel (21, 31, 41, 51) und/oder dem Tastergehäuse (23, 33, 43, 53) verbindbar ist und jede dieser einer Feder (80, 81) zugeordnete Aufnahme (82A-C, 83A-C) eine andere Entfernung zur Drehachse (26) des Tasters (2 - 5) aufweist.

12. Fußsteuerung (1) nach Anspruch 11,
**dadurch gekennzeichnet, daß**
zwei Federn (80, 81) den Tasterdeckel (21, 31, 41, 51) vorspannen und jeder Feder (80, 81) mehr als eine Aufnahme (82A-C, 83A-C), bevorzugt drei bis fünf Aufnahmen, zugeordnet sind.
